# EUROPÄISCHE PATENTSCHRIFT

(11) **EP 1 090 602 B1**
(45) Veröffentlichungstag und Bekanntmachung des Hinweises auf die Patenterteilung: **26.04.2006**
(21) Anmeldenummer: 00120420.5
(22) Anmeldetag: 18.09.2000
(51) Int. Cl.: A61C 1/00

(54) **Zahnmedizinische Einrichtung**
Dental device
Dispositif dentaire

(30) Priorität: 08.10.1999 DE 19948620
(43) Veröffentlichungstag der Anmeldung: 11.04.2001
(73) Patentinhaber: Kaltenbach & Voigt GmbH, 88400 Biberach / Riss (DE)
(72) Erfinder: Steddin, Sven-Dieter, Dr., 88400 Biberach (DE)
(74) Vertreter: Schmidt-Evers, Jürgen

(56) Entgegenhaltungen:
- DE-U- 29 621 939
- US-A- 5 230 623
- US-A- 5 828 197

## Beschreibung

Die vorliegende Erfindung betrifft eine zahnmedizinische Einrichtung sowie ein Verfahren zum effektiven Steuern dieser Einrichtung.

Bei modernen medizinischen Einrichtungen muß oftmals eine Vielzahl von Funktionen der medizinischen Gerätschaften mit Hilfe unterschiedlicher Steuereinrichtungen angesteuert werden. Beispielsweise werden im Laufe einer komplexen Behandlung mit mehreren aufeinanderfolgenden Behandlungsschritten die Betriebsparameter der unterschiedlichen Gerätschaften für jeden Behandlungsschritt speziell gewählt. Dabei sind den Gerätschaften sowohl die Behandlungsinstrumente selbst als auch beispielsweise der Patientenstuhl oder weitere Hilfseinrichtungen zuzurechnen.

Mehrere Veröffentlichungen befassen sich mit einem einfachen und effektiven Ansteuern der unterschiedlichen Funktionen von medizinischen Einrichtungen. Dabei ist insbesondere bei zahnmedizinischen Einrichtungen der Fußschalter die nach wie vor am häufigsten verwendete Eingabevorrichtung. Weiterentwicklungen des klassischen Fußschalters sind beispielsweise aus der EP 0 525 539 A2 bekannt. Dabei werden über Drucksensoren die Belastungen der Oberfläche des Fußschalters in primäre elektrische Signale umgewandelt, die mittels einer Signalauswerteelektronik in Steuersignale umgewandelt und auf eine zentrale Steuereinheit übertragen werden. Auf diese Weise wird eine zahnärztliche Behandlungseinrichtung mit einem Patientenstuhl und mehreren Dentalgeräten gesteuert. Eine in der EP 0 455 852 B1 beschriebene zahnärztliche Einrichtung weist ferner eine Anzeigeeinrichtung auf, auf der sämtliche wichtigen Funktionen der Einrichtung dargestellt werden. Das Bedienen der unterschiedlichen Funktionen wird lediglich durch ein einziges Eingabeinstrument, nämlich einen in zwei Freiheitsgraden verstellbaren Fußschalter ermöglicht. Auch die in der EP 0 391 967 B1 offenbarte zahnärztliche Vorrichtung ermöglicht eine Steuerung sämtlicher Operationen und Hilfsfunktionen über lediglich einen einzigen Fußschalter.

Da zahnärztliche Behandlungsplätze immer komplexer und vielfältiger aufgebaut werden, ist die Steuerung sämtlicher Funktionen eines Behandlungsplatzes durch einen einzigen Fußschalter immer schwieriger zu bewerkstelligen. Die Möglichkeiten eines Fußschalters als Steuereinrichtung sind nämlich aufgrund der Bedienung mit den Füßen und der zur Verfügung stehenden Freiheitsgrade begrenzt. Oftmals ist es erforderlich, zum Ändern eines einzigen Parameters eine Reihe von Untermenüs anzuwählen, bis schließlich die gewünschte Änderung durchgeführt werden kann. Die hierfür benötigten zahlreichen Eingabeschritte und der damit verbundene Zeitaufwand können allerdings nicht unbedingt als sehr bedienungsfreundlich angesehen werden.

Die in der DE 296 21 939 U1 beschriebene medizinischen Einrichtung weist daher eine Eingabevorrichtung auf, welche ein Zeigegerät, vorzugsweise ein Mauspad mit Druck-und Positionssensorik enthält, über das von Hand die verschiedenen Funktionen der Einrichtung, deren entsprechende Parameter auf einer Anzeigeeinheit dargestellt werden, gesteuert werden. Als weiteres Eingabegerät wird ein Fußschalter verwendet, über den die Steuerung des gerade verwendeten Behandlungsinstruments erfolgt.

Aus der Neurochirurgie ist schließlich die Positions-Erfassung von medizinischen Behandlungsinstrumenten bekannt. Bei in diesem Bereich durchgeführten Operationen ist es oftmals unerläßlich, die exakte Position des Behandlungsinstruments relativ zur Anatomie des Patienten zu kennen, um die Operation auch tatsächlich mit hoher Genauigkeit an der zu behandelnden Stelle durchführen zu können. Neuchirugische Behandlungseinrichtungen weisen daher grundsätzlich ein Behandlungsinstrument sowie eine Anzeigeeinheit und Mittel zum Erfassen der Position des Behandlungsinstruments auf, wobei während der Behandlung permanent die Position des Behandlungsinstruments relativ zur Anatomie des Patienten auf der Anzeigeeinheit dargestellt wird. Das Überwachen und Darstellen der Position des Behandlungsinstruments erfolgt über die Steuersoftware der Behandlungseinrichtung.

In der US 5,230,623 wird vorgeschlagen, die Steuersoftware einer neurochirurgischen Behandlungseinrichtung derart zu erweitern, daß die von dem Behandlungsinstrument permanent abgegebenen Steuersignale vorübergehend auch zum Bedienen eines auf der Anzeigeeinheit dargestellten Menüs verwendet werden. Auf diese Weise können unterschiedliche Funktionen der Behandlungseinrichtung angewählt werden. Da die hardware- und softwaremäßigen Voraussetzungen zum Erfassen der Position des Behandlungsinstruments bzw. zum Umsetzen der räumlichen Position in Steuersignale ohnehin vorhanden sind, müssen dafür lediglich geringfügige Änderungen in der Steuersoftware vorgenommen werden.

Im Gegensatz dazu hat die vorliegende Erfindung zum Ziel, eine zahnmedizinische Einrichtung hardwaremäßig derart zu erweitern, daß herkömmliche Behandlungsinstrumente zusätzlich auch als Eingabevorrichtungen zum Steuern der gesamten Einrichtung verwendet werden können.

Es ist somit Aufgabe der vorliegenden Erfindung eine zahnmedizinische Einrichtung anzugeben, bei der die zahlreichen Funktionen der Gerätschaften auf eine neue und möglichst benutzerfreundliche und effektive Weise angesteuert werden können.

Die Aufgabe wird durch eine Einrichtung, welche die Merkmale des Anspruches 1 aufweist, gelöst.

Die Einrichtung weist mindestens ein Behandlungsinstrument, eine zentrale Steuereinrichtung sowie eine Anzeigeeinheit auf, auf der beispielsweise unterschiedliche Betriebsparameter und Funktionen der Einrichtung dargestellt werden. Erfindungsgemäß weist die Einrichtung ferner Mittel zum Erfassen der räumlichen Position des Behandlungsinstrumentes auf. Zum Ändern bestimmter auf der Anzeigeeinheit dargestellter Betriebsparameter oder zum Anwählen einer Funktion wird dann in einem Steuermodus die räumliche Position des Behandlungsinstruments in Steuersignale für einen auf der Anzeigeeinheit bewegbaren Zeiger umgesetzt. Die Mittel zum Erfassen der räumlichen Position des Behandlungsinstruments sind ferner so ausgebildet, daß sie auch eine räumliche Orientierung des Behandlungsinstruments erfassen, wobei die Steuereinheit die erfaßte räumliche Orientierung und/oder Änderungen in der räumlichen Orientierung ebenfalls für die Erzeugung von Steuersignalen berücksichtigt.

Das Behandlungsinstrument übernimmt somit während des Steuermodus die Funktion einer Einabevorrichtung, wobei die Funktionsweise derjenigen einer Computer-Maus entspricht, so daß die gesamte zahnmedizinische Einrichtung berührungslos und komfortabel bedient werden kann. Zum Aufrufen einer bestimmten Funktion oder zum Ändern eines Betriebsparameters ist es daher nicht mehr notwendig, das Behandlungsinstrument abzulegen, um ein spezielles Eingabeeinstrument zu betätigen. Dadurch wird auch die Gefahr vermieden, ein oftmals sehr schwierig zu reinigendes bzw. zu desinfizierendes Eingabeinstrument - beispielsweise eine Tastatur - zu kontaminieren.

Unter dem oben erwähnten Behandlungsinstrument werden dabei sämtliche Instrumente eines zahnmedizinischen Behandlungsplatzes verstanden. Neben dem üblichen Bohrer kann es sich beispielsweise um eine intraorale Kamera, eine Meßsonde oder dergleichen handeln. Wesentlich dabei ist, daß eine Positionsbestimmung dieser Instrumente aufgrund ihrer Funktionsweise von Haus aus nicht möglich bzw. notwendig ist, daß diese aber entsprechend der Erfindung nun zusätzlich die Funktion eines Eingabeinstruments erfüllen können.

Weiterbildungen der Erfindung sind Gegenstand der Unteransprüche. So können zur Ermittlung der räumlichen Position des Zeigeinstruments alle bekannten Technologien verwendet werden, z.B. die in den Offenlegungsschriften EP 0 789 320 A2, EP 0 526 015 A1 und DE 38 38 605 A1 beschriebene Positionsbestimmung mittels Ulltraschallimpulsen. Vorzugsweise wird die Position jedoch durch Emittieren und Empfangen von Infrarotstrahlung erfaßt. Beispielsweise kann dafür an der Anzeigeeinheit eine Infrarot-Lichtquelle vorgesehen sein. Die von dieser Lichtquelle emittierte Strahlung wird dann von einem an dem Behandlungsinstrument angebrachten passiven Marker (Reflektor) reflektiert und von einem wiederum an der Anzeigeeinheit vorgesehenen optischen Sensor - beispielsweise einer Kamera oder einer 4-Felder-Photodiode - erfaßt. Alternativ dazu kann auch eine aktive Infrarot-Lichtquelle direkt an dem Behandlungsinstrument angeordnet werden, wobei dann auf die Lichtquellen an der Anzeigeeinheit verzichtet werden kann. Die von dem optischen Sensor detektierten Koordinaten der Lichtquelle bzw. des Reflektors an dem Behandlungsinstrument werden dann in kartesische Koordinaten auf der Anzeigeeinheit umgerechnet, wodurch die Position des Zeigers durch einfaches Bewegen des Behandlungsinstruments verändert werden kann.

Alternativ dazu besteht die Möglichkeit, mit den Markern an dem Behandlungsinstrument ein zeitlich veränderbares Signal zu erzeugen, welches von der Kamera detektiert und als zusätzliches Steuersignal interpretiert wird. Dieses zeitlich veränderbare Signal kann beispielsweise durch unterschiedliche An/Aus-Sequenzen der Lichtquelle bzw. der Reflexionseigenschaften des Reflektors gebildet werden. Das Auslösen derartiger Sequenzen kann beispielsweise über einen an dem Instrument angebrachten Schalter erfolgen. Ebenfalls läßt sich über ein zeitlich veränderbares Signal der Störabstand des Systems verbessern, da der Einfluß anderer Infrarotquellen (z.B. Reflexionen an einer Lampe oder einem Knopf) auf diese Weise minimiert wird.

Da erfindungsgemäß das Behandlungsinstrument die Funktion einer Maus übernehmen soll, ist es vorteilhaft, wenn neben den Signalen zum Bewegen des Zeigers weitere Steuersignale, die beispielsweise dem Drücken einer linken oder rechen Maustaste entsprechen, erzeugt werden können. Hierfür kann beispielsweise die zusätzliche Verwendung eines Fußschalters vorgesehen sein.

Vorteilhaft soll parallel zu der Verwendung des Behandlungsinstruments als Eingabegerät immer noch die Verwendung einer herkömmlichen Maus möglich sein.

Im folgenden soll die Erfindung anhand der beiliegenden Zeichnungen näher erläutert werden. Es zeigen:
Fig. 1 ein Ausführungsbeispiel einer erfindungsgemäßen zahnmedizinischen Einrichtung;
Fig. 2a bis 2c schematische Darstellungen zur Erzeugung zusätzlicher Steuersignale bei der Verwendung von zwei Markern; und
Fig. 3a bis 3c schematische Darstellungen unterschiedlicher Markeranordnungen.

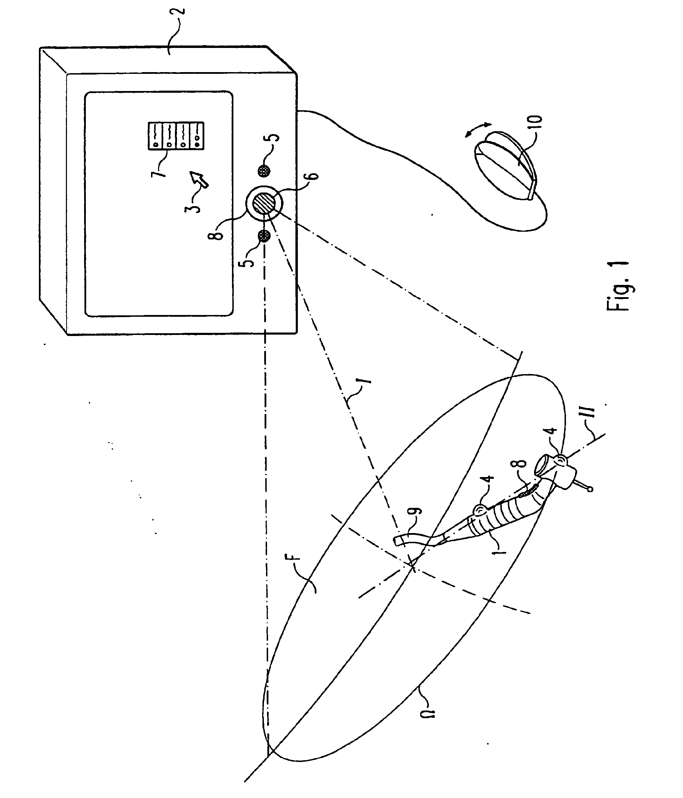

Der allgemeine Aufbau eines zahnmedizinischen Behandlungsplatzes ist aus dem Stand der Technik bereits hinlänglich bekannt. In Fig. 1 werden daher lediglich die für die Erfindung wesentlichen Elemente dargestellt. Diese sind ein Behandlungsinstrument 1 - im dargestellten Beispiel ein Bohrer - sowie eine Anzeigeeinheit (Monitor) 2 und eine nicht dargestellte zentrale Steuereinrichtung. Auf dem Bildschirm des Monitors 2 können beispielsweise die Betriebsdaten des jeweils im Einsatz befindlichen Behandlungsinstrumentes 1, Wartungshinweise, Patientendaten in Text und/oder Bild und verschiedene einstellbare und fest vorgegebene Funktionen des Behandlungsplatzes, sowie beliebige Computerprogramme angezeigt werden. Um eine übersichtliche Darstellung zu ermöglichen, können mit Hilfe eines Menüs 7 unterschiedliche Funktionen aufgerufen werden. Dabei ist auch die Möglichkeit gegeben, für eine in mehreren Schritten durchzuführende Behandlung für jeden Schritt bereits zuvor einprogrammierte Betriebsparameter und Funktionen anzuwählen. Das Anwählen bestimmter Funktionen und das Verändern von Betriebsparametern erfolgt durch Anwählen der entsprechenden Symbole auf der Bildschirmoberfläche durch den Zeiger 3.

Erfindungsgemäß wird der Zeiger 3 in einem Steuermodus mit Hilfe des als Eingabevorrichtung dienenden Behandlungsinstruments 1 angesteuert. Zu diesem Zweck sind an dem Behandlungsinstrument 2 zwei passive Infrarotmarker (Reflektoren) 4 angebracht. An der Unterseite des Bildschirms 2 befinden sich zwei Infrarot-Lichtquellen 5 - beispielsweise IR-LEDs - über welche das Instrument 1 beleuchtet wird. Die Reflexionen des Infrarotlichtes durch die beiden auf dem Behandlungsinstrument 1 angeordneten Marker 4 wird durch eine ebenfalls an der Unterseite des Bildschirms 2 angebrachte infrarot-empfindliche Kamera 6 detektiert. Vorzugsweise wird die Kamera 6 mit einem Tageslichtsperrfilter 8 versehen, um den Störabstand gegenüber Umwelteinflüssen zu vergrößern. Der von der Kamera 6 erfaßbare Raumwinkel Ω ist mit dem von den beiden Lichtquellen 5 bestrahlten Bereich identisch.

Im Folgenden soll die Funktionsweise der Kamera 6 näher erläutert werden. Durch die Kamera 6 werden die beiden Marker 4 detektiert und deren auf die optische Achse I der Kamera 6 bezogene Kugelkoordinaten berechnet. Dabei kann der Abstand des Behandlungsinstruments 1 von der Kamera 6 innerhalb bestimmter Grenzen beliebig gewählt werden. Die berechneten Kugelkoordinaten werden dann von der Steuereinheit auf kartesische Koordinaten für die Bildschirmoberfläche umgerechnet und der Zeiger 3 in der entsprechenden Position dargestellt. Auf diese Weise kann während des Steuermodus durch einfaches Bewegen des Behandlungsinstrumentes 1 der Zeiger 3 über die Oberfläche des Bildschirms 2 bewegt werden. Die Empfindlichkeit bei der Umsetzung auf die kartesischen Koordinaten ist dabei umgekehrt proportional zum Abstand der beiden Marker 4 von der Kamera 6 und kann somit von dem Anwender gezielt variiert werden. Um schnelle und damit auch grobe Bewegungen des Zeigers 3 durchzuführen, muß sich das Behandlungsinstrument 1 in einem nur geringen Abstand von der Kamera 6 befinden, während bei größerem Abstand langsame aber dafür genaue Bewegungen des Zeigers 3 erzielt werden können.

Die Bewegung des Zeigers 3 durch das Behandlungsinstrument 1 kann absolut oder relativ erfolgen. Im ersten Fall entspricht jeder Position des Behandlungsinstruments 1 in dem von der Kamera 6 erfaßten Raumbereich Ω exakt eine Koordinate des Zeigers 3 auf dem Bildschirm. Im zweiten Fall wird die Position des Zeigers 3 durch die Bewegung des Behandlungsinstruments 1 relativ zur Position des Zeigers 3 zu Beginn des Steuermodus verändert. Dieser Fall entspricht dem Verhalten einer üblichen Maus. Das zwischenzeitliche Aussetzen der Zeigerbewegung, welches bei der normalen Computer-Maus durch Anheben erfolgt, kann durch einen zusätzlichen Schalter oder durch Ignorieren von sehr schnellen Bewegungen simuliert werden.

Alternativ zu der Kamera 6 könnten als optischer Sensor auch eine 4-Felder-Photodiode bzw. mehrere verteilt angeordnete Photodioden verwendet werden. Mit Hilfe einer Schwerpunktberechnung werden dann die erfaßten Signale in entsprechende Koordinaten umgesetzt.

Um beispielsweise eine der Funktionen des auf dem Bildschirm 2 dargestellten Menüs 7 anwählen zu können, muß ferner auch die Möglichkeit gegeben sein, zusätzliche Steuersignale, die beispielsweise einem Maus-Klick entsprechen, erzeugen zu können. Hierfür kann an dem Behandlungsinstrument 1 ein zusätzlicher Schalter 8 vorgesehen sein. Bei Betätigen dieses Schalters 8 werden dann z.B. entsprechende Steuersignale über den Verbindungsschlauch 9 an die zentrale Steuereinrichtung weitergeleitet. Alternativ dazu könnte auch vorgesehen sein, daß bei Betätigen des Schalters 8 ein zeitlich veränderbares Signal erzeugt wird, welches von der Kamera 6 detektiert und als entsprechender Maus-Klick interpretiert wird. Beispielsweise könnte durch Änderungen der Reflexionseigenschaften der Marker 4 mit Hilfe von Polarisationsfolien eine zeitliche An/Aus-Sequenz erzeugt werden.

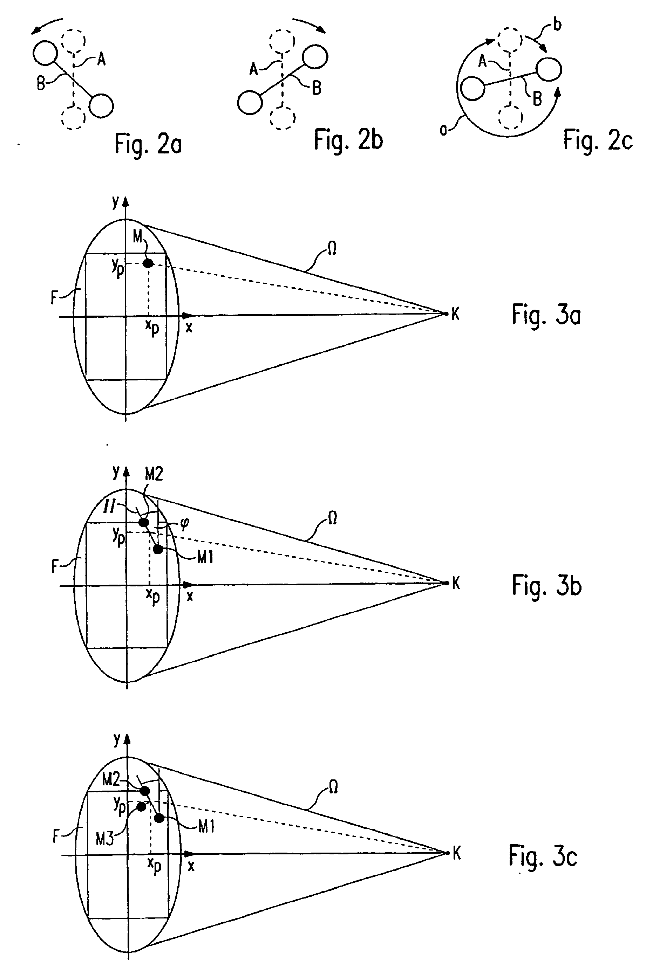

Weist der Schalter 8 zwei anzuwählende Schaltzustände auf, so können damit in einfacher Weise ein rechter und ein linker Maus-Klick nachgeahmt werden. Bietet der Schalter 8 jedoch nur die Möglichkeit eines einzigen Schaltzustandes, so kann zunächst lediglich ein Steuersignal erzeugt werden. In diesem Fall kann die Orientierung des Behandlungsinstruments 1 zusätzlich berücksichtigt werden. Zur Berechnung einer kartesischen Koordinate für den Zeiger 3 ist nämlich zunächst lediglich ein einziger Marker 4 ausreichend. Durch den zweiten Marker wird allerdings eine Gerade II definiert, deren vertikale Lage im Raum durch die zentrale Steuereinheit berechenbar ist. Die Ausrichtung der Projektion dieser Geraden II in eine senkrecht zur optischen Achse 1 der Kamera 6 stehenden Fläche F kann dann für zusätzliche Steuerfunktionen ausgewertet werden. Beispielsweise kann aufgrund der Ausrichtung der Geraden II zwischen einem rechten oder linken Maus-Klick unterschieden werden. Vorzugsweise wird jedoch nicht die absolute Orientierung des Behandlungsinstruments 1 sondern eine Änderung der Orientierung zum Erzeugen der zusätzlichen Steuersignale verwendet. Beispielsweise kann vorgesehen sein, daß nach Betätigen des Schalters 8 unterschieden wird, ob das Behandlungsinstrument 1 im Uhrzeigersinn oder gegen den Uhrzeigersinn gedreht wird, was dann jeweils dem rechten oder dem linken Maus-Klick entspricht. Dies ist in den Figuren 2a und 2b dargestellt, wobei A jeweils die Orientierung der Instrumenten-Achse vor und B deren Orientierung nach der Drehung bezeichnet. Dementsprechend wird die in Fig. 2a dargestellte Drehung entgegen dem Uhrzeigersinn einem Klick einer linken Maustaste und die in Fig. 2b dargestellte Drehung einem rechten Maus-Klick zugeordnet.

Da allerdings die Gefahr besteht, daß beim Drehen des Behandlungsinstruments 1 auch der Zeiger 3 verschoben wird, kann vorgesehen sein, daß während einer Betätigung des Schalters 8 der Zeiger 3 nicht mehr bewegt wird, sondern nur noch zwischen den unterschiedlichen Drehrichtungen des Instruments 1 unterschieden wird. Wird das Instrument 1 mit drei Markern 4 ausgestattet, so läßt sich auch die absolute Lage des Instruments 1 (nach links oder rechts gedreht) verwenden und einem entsprechenden Klick der linken oder rechten Maustaste zuordnen.

Eine weitere Möglichkeit besteht darin, zum Erzeugen der zusätzlichen Steuersignale einen mit der zentralen Steuereinheit und dem Monitor 2 verbundenen Fußschalter 10 zu verwenden. In diesem Fall kann auf den Schalter 8 an dem Behandlungsinstrument 1 verzichtet werden. Weist der Fußschalter 10 lediglich eine Schaltfläche auf, so können wie eben beschrieben mit Hilfe von Drehbewegungen des Behandlungsinstruments 1 unterschiedliche Steuersignale erzeugt werden.

Der Fußschalter 10 bzw. der Schalter 8 am Behandlungsinstrument 1 kann auch dafür verwendet werden, den Steuermodus zu aktivieren. Alternativ dazu kann festgelegt werden, daß der Steuermodus aktiviert wird, sobald Signale der Marker 4 über die Dauer eines vordefinierten und einstellbaren Zeitfensters von der Kamera 6 detektiert werden. Ist dieser Steuermodus aktiviert, so kann der Fußschalter 10 für die Erzeugung der zusätzlichen Steuersignale verwendet werden. Nach Beenden des Steuermodus nimmt er wieder seine gewohnten, vom jeweiligen aktivierten Instrument abhängigen Funktionen (Anlasser usw.) war.

Anstelle der Verwendung von passiven Markern 4 können auch aktive Lichtquellen an dem Behandlungsinstrument 1 vorgesehen werden, wobei dann auf die Infrarot-Lichtquellen 5 an der Anzeigeeinheit 2 verzichtet werden kann. Die Marker werden dann vorzugsweise in die Kupplung zwischen dem aufsteckbaren Instrument und dem Versorgungsschlauch integriert, da zum einen dann eine entsprechende Stromversorgung zur Verfügung steht und da zum anderen die Instrumente selbst nicht neu konstruiert werden müssen. Bei passiven Markern kann hingegen auch eine separate Haltevorrichtung verwendet werden, beispielsweise in Form eines Klemmringes oder dergleichen, die auf das Behandlungsinstrument aufgesteckt oder aufgeschoben wird.

Ferner können anstelle von einem oder zwei Markern auch drei Marker an dem Instrument 1 angebracht sein, was dann von Vorteil ist, wenn die exakte 3-dimensionale Anordnung des Behandlungsinstruments 1 zum Erzeugen zusätzlicher Steuersignale ausgenutzt werden soll. Befinden sich drei Marker in einer Dreiecksanordnung (wobei es sich dabei nicht um ein gleichseitiges Dreieck handeln darf) so kann die absolute Orientierung des Instrumentes 1 im Raum bestimmt werden. Im Gegensatz dazu ergeben sich bei der Verwendung von lediglich zwei Markern Mehrdeutigkeiten, da um 180° gedrehte Positionen nicht unterschieden werden können. Wie beispielhaft in Fig. 2c dargestellt ist, besteht die Gefahr, daß sich aus der Endposition B nicht mehr ableiten läßt, ob eine Linksdrehung a oder Rechtsdrehung b erfolgte.

Bei der Verwendung von drei Markern oder von zwei räumlich getrennten Bildsensoren besteht ferner die Möglichkeit, die 3. Dimension zur Bedienung der Behandlungseinheit zu verwenden. Beispielsweise können von der Entfernung des Behandlungsinstruments 1 von der Anzeigeeinheit 2 abhängig automatisch unterschiedliche Funktionen des Arbeitsplatzes gesteuert werden; befindet sich das Behandlungsinstrument 1 nahe an der Anzeigeeinheit 2 kann z.B. der Patientenstuhl gesteuert werden, bei mittlerer Entfernung wird automatisch eine Steuermenü für eine Kameraaufnahme geöffnet und bei großem Abstand wird eine Menüoberfläche für die Leistungserfassung dargestellt. Auf diese Weise würde das Umschalten zwischen verschiedenen Menüoberflächen oder das Blättern innerhalb eines Programmes vereinfacht.

Um den Maus-Klicks entsprechende Steuersignale oder aber auch darüber hinaus weitere Steuersignale zu erzeugen, kann an der Behandlungseinheit ferner ein Spracherkennungsmodul vorgesehen sein.

Wurde die entsprechende Funktion auf der Bildschirmoberfläche mit Hilfe des als Eingabeinstrument fungierenden Behandlungsinstruments 1 durchgeführt, kann der Steuermodus deaktiviert werden und das Behandlungsinstrument 1 wieder in seiner ursprünglichen Funktion in gewohnter Weise verwendet werden. Enthält die zahnmedizinische Einrichtung mehrere Behandlungsinstrumente, so kann jedes dieser Instrumente während des Steuermodus als Eingabeinstrument fungieren. Weisen die Instrumente passive Marker auf, so werden die Marker der sich in der Instrumentenablage befindenden Instrumente ohnehin verdeckt, so daß diese nicht zu einem störenden Signal führen können. Sind jeweils aktive Marker vorhanden, so können in der Instrumentenablage Sensoren angebracht werden, die lediglich das Erzeugen von Infrarotsignalen bei Markern eines entnommenen Instruments ermöglichen.

Somit kann die gesamte zahnmedizinische Einrichtung in einfacher und effektiver Weise berührungslos gesteuert werden. Ferner sind auch keine zusätzlichen Eingabegeräte notwendig, die der Zahnarzt mit seinen möglicherweise kontaminierten Fingern betätigen muß, da er das Behandlungsinstrument 1 kontinuierlich in der Hand behalten kann. Bei der Verwendung von passiven Markern können die Behandlungsinstrumente 1 auch ohne größere Schwierigkeiten gereinigt und sterilisiert werden. Vorzugsweise weist die zahnmedizinische Einrichtung allerdings auch eine herkömmliche Maus auf, die parallel zu dem Behandlungsinstrument 1 als Eingabeinstrument verwendet werden kann.

Im folgenden sollen anhand der Figuren 3a bis 3c kurz die sich bei unterschiedlichen Markeranordnungen ergebenden Möglichkeiten zum Erzeugen von zusätzlichen Steuersignalen kurz erläutert werden.

Fig. 3a zeigt eine Skizze für die Verwendung eines einzelnen aktiven oder passiven Markers M an dem Behandlungsinstrument. Die Detektierung dieses Markers M erlaubt die Festlegung von dessen Position innerhalb der in den Blickkegel Ω der Kamera K gelegten Fläche F. Die Position des Markers M kann dann in entsprechende Bildschirmkoordinaten xₚ und yₚ für den Zeiger auf dem Bildschirm umgerechnet werden. Da lediglich ein Marker M vorhanden ist, können zusätzliche Steuersignale zum Simulieren von Maus-Klicks lediglich durch ein zusätzliches Eingabeinstrument - beispielsweise den Fußschalter - oder durch Erzeugen von speziellen Lichtsequenzen erzeugt werden.

Bei dem in Fig. 3b dargestellten Beispiel mit zwei Markern M1 und M2 kann zusätzlich zur Positionsbestimmung der beiden Marker auch noch die Orientierung der Verbindungsgeraden II relativ zur x- oder zur y-Achse ermittelt werden. Der dabei bestimmt Winkel ϕ kann wie zuvor beschrieben - evtl. mit Hilfe eines weiteren Schalters oder Fußschalters - zur Erzeugung weiterer Steuersignale verwendet werden. Für die Koordinaten xₚ und yₚ des Zeigers auf der Bildschirmoberfläche wird vorzugsweise der Mittelpunkt zwischen den beiden Markern M1 und M2 gewählt. Falls die beiden Marker M1 und M2 baugleich sind, kann eine Vertauschung ihrer Anordnung, die sich beispielsweise bei einer Drehung um 180° ergibt, allerdings nicht erfaßt werden, so daß sich möglicherweise Mehrdeutigkeiten ergeben können. Wie bereits erwähnt wurde, besitzt diese Anordnung daher den Nachteil, daß sich die Drehung des Instruments nicht eindeutig bestimmen läßt, da bei einer Drehung aus jeder beliebigen Position um 180° exakt das gleiche Abbild der Markeranordnung entsteht. Es besteht aber die Möglichkeit, dieses Problem zu umgehen, indem die Marker so ausgebildet oder betrieben werden, daß sie sich eindeutig identifizieren lassen. Dies kann beispielsweise über eine unterschiedliche Form der Marker oder durch Modulation der von den Markern emittierten Lichtsignale erfolgen.

Die zuvor beschriebenen Mehrdeutigkeiten können schließlich bei dem in Fig. 2c dargestellten Beispiel durch die Verwendung von drei Matkern M1 bis M3 ausgeschlossen werden, da nun die exakte Orientierung und Position des Instruments im Raum bestimmt werden kann. Auch hier bietet sich die Möglichkeit, unterschiedliche Orientierungen zur Erzeugung von zusätzlichen Steuerungsfunktionen zu verwenden. Da bei einer zahnmedizinischen Einrichtung in der Regel ohnehin ein Fußschalter vorhanden ist, ist allerdings der Verwendung von einem einzigen oder zwei Markern der Vorzug zu geben.

## Patentansprüche

1. Zahnmedizinische Einrichtung mit
mindestens einem Behandlungsinstrument (1), einer zentralen Steuereinrichtung und einer Anzeigeeinheit (2),
**dadurch gekennzeichnet,**
**daß** die Einrichtung Mittel (4, 5, 6) zum Erfassen der räumlichen Position des Behandlungsinstruments (1) aufweist, wobei in einem Steuermodus die erfaßte räumliche Position des Behandlungsinstruments (1) von der Steuereinrichtung in Koordinaten zum Steuern eines Zeigers (3) auf der Anzeigeeinheit (2) umsetzbar ist, wobei die Mittel (4, 5, 6) zum Erfassen der räumlichen Position des Behandlungsinstruments (1) ferner so ausgebildet sind, daß sie auch eine räumliche Orientierung des Behandlungsinstruments (1) erfassen, wobei die Steuereinheit die erfaßte räumliche Orientierung und/oder Änderungen in der räumlichen Orientierung ebenfalls für die Erzeugung von Steuersignalen berücksichtigt.

2. Zahnmedizinische Einrichtung nach Anspruch 1,
**dadurch gekennzeichnet,**
**daß** die Mittel (4, 5, 6) zum Erfassen der räumlichen Position des Behandlungsinstruments (1) an der Anzeigeeinheit (2) mindestens eine Infrarot-Lichtquelle (5) sowie einen optischen Sensor (6) und mindestens einen an dem Behandlungsinstrument angebrachten Reflektor (4) zum Reflektieren der von der mindestens einen Infrarot-Lichtquelle (5) emittierten Strahlung aufweisen.

3. Zahnmedizinische Einrichtung nach Anspruch 1,
**dadurch gekennzeichnet,**
**daß** die Mittel (4, 5, 6) zum Erfassen der räumlichen Position des Behandlungsinstruments (1) mindestens eine an dem Behandlungsinstrument (1) angebrachte Infrarot-Lichtquelle sowie einen an der Anzeigeeinheit (2) angeordneten optischen Sensor (6) aufweisen.

4. Zahnmedizinische Einrichtung nach Anspruch 2 oder 3,
**dadurch gekennzeichnet,**
**daß** es sich bei dem optischen Sensor um eine infrarot-empfindliche Kamera (6) handelt.

5. Zahnmedizinische Einrichtung nach Anspruch 2 oder 3,
**dadurch gekennzeichnet,**
**daß** es sich bei dem optischen Sensor um eine 4-Felder-Photodiode handelt.

6. Zahnmedizinische Einrichtung nach einem der vorherigen Ansprüche,
**dadurch gekennzeichnet,**
**daß** diese Mittel zum Erzeugen zusätzlicher Steuersignale für den Zeiger (3) während des Steuermodus aufweist.

7. Zahnmedizinische Einrichtung nach Anspruch 6,
**dadurch gekennzeichnet,**
**daß** diese zum Erzeugen der zusätzlichen Steuersignale eine an dem Behandlungsinstrument (1) angebrachte Schaltervorrichtung (8) aufweist.

8. Zahnmedizinische Einrichtung nach Anspruch 6,
**dadurch gekennzeichnet,**
**daß** diese zum Erzeugen der zusätzlichen Steuersignale einen Fußschalter (10) aufweist.

9. Zahnmedizinische Einrichtung nach einem der vorherigen Ansprüche,
**dadurch gekennzeichnet,**
**daß** die Einrichtung zur Erzeugung weiterer Steuersignale eine **Spracherkennungsmodul aufweist.**

10. Zahnmedizinische Einrichtung nach einem der vorherigen Ansprüche,
**dadurch gekennzeichnet,**
**daß** die Einrichtung einen Schalter (8, 10) zum Aktivieren des Steuermodus aufweist.

11. Verfahren zum Steuern einer zahnmedizinischen Einrichtung,
die mindestens ein Behandlungsinstrument (1), eine zentrale Steuereinrichtung und eine Anzeigeeinheit (2) aufweist,
**dadurch gekennzeichnet,**
**daß** in einem Steuermodus die räumliche Position des Behandlungsinstruments (1) erfaßt und in Koordinaten zum Steuern und Bedienen eines Zeigers (3) auf der Anzeigeeinheit (2) umgesetzt wird,
wobei in dem Steuermodus zusätzlich zur Position auch eine Orientierung des Behandlungsinstruments (1) erfaßt wird und die Orientierung und/oder Änderungen der Orientierung in zusätzliche Steuersignale umgesetzt werden.

## Revendications

1. Dispositif de traitement dentaire, comportant au moins un instrument de traitement (1), un dispositif de commande central et une unité d'affichage (2), **caractérisé en ce que** ledit dispositif comporte des moyens (4, 5, 6) destinés à détecter la position dans l'espace de l'instrument de traitement (1), la position dans l'espace détectée de l'instrument de traitement (1) pouvant être transformée, dans un mode de commande, en coordonnées pour la commande d'un pointeur (3) sur l'unité d'affichage (2), les moyens (4, 5, 6) destinés à détecter la position dans l'espace de l'instrument de traitement (1) étant réalisés en outre de telle sorte qu'ils détectent également une orientation dans l'espace de l'instrument de traitement (1), le dispositif de commande tenant également compte de l'orientation dans l'espace détectée et/ou des variations de l'orientation dans l'espace pour générer des signaux de commande.

2. Dispositif de traitement dentaire selon la revendication 1, **caractérisé en ce que** les moyens (4, 5, 6) destinés à détecter la position dans l'espace de l'instrument de traitement (1) comportent sur l'unité d'affichage (2) au moins une source lumineuse infrarouge (5), ainsi qu'un capteur optique (6) et au moins un réflecteur (4), agencé sur l'instrument de traitement (1) en vue de réfléchir le rayonnement émis par ladite au moins une source lumineuse infrarouge (5).

3. Dispositif de traitement dentaire selon la revendication 1, **caractérisé en ce que** les moyens (4, 5, 6) destinés à détecter la position dans l'espace de l'instrument de traitement (1) comportent au moins une source lumineuse infrarouge agencée sur l'instrument de traitement (1), ainsi qu'un capteur optique (6) agencé sur l'unité d'affichage (2).

4. Dispositif de traitement dentaire selon la revendication 2 ou 3, **caractérisé en ce que** le capteur optique est une caméra (6) sensible à l'infrarouge.

5. Dispositif de traitement dentaire selon la revendication 2 ou 3, **caractérisé en ce que** le capteur optique est une photodiode à 4 champs.

6. Dispositif de traitement dentaire selon l'une quelconque des revendications précédentes, **caractérisé en ce que** ledit dispositif comporte des moyens destinés à générer des signaux de commande supplémentaires pour le pointeur (3) pendant le mode de commande.

7. Dispositif de traitement dentaire selon la revendication 6, **caractérisé en ce que** ledit dispositif, pour générer les signaux de commande supplémentaires, comporte un dispositif de commutation (8) agencé sur l'instrument de traitement (1).

8. Dispositif de traitement dentaire selon la revendication 6, **caractérisé en ce que** ledit dispositif, pour générer les signaux de commande supplémentaires, comporte un commutateur à commande au pied (10).

9. Dispositif de traitement dentaire selon l'une quelconque des revendications précédentes, **caractérisé en ce que** le dispositif, pour générer d'autres signaux de commande, comporte un module de reconnaissance vocale.

10. Dispositif de traitement dentaire selon l'une quelconque des revendications précédentes, **caractérisé en ce que** le dispositif comporte un interrupteur (8, 10) destiné à activer un mode de commande.

11. Procédé de commande d'un dispositif de traitement dentaire comportant au moins un instrument de traitement (1), un dispositif de commande central et une unité d'affichage (2), **caractérisé en ce que**, dans un mode de commande, la position dans l'espace de l'instrument de traitement (1) est détectée et est transformée en coordonnées pour commander et manoeuvrer un pointeur (3) sur l'unité d'affichage (2), une orientation dans l'espace de l'instrument de traitement (1) étant détectée dans le mode de commande en plus de la position, et l'orientation et/ou des variations de l'orientation étant transformées en signaux de commande supplémentaires.

## Claims

1. Dental device
having at least one treatment instrument (1), a central control device and a display unit (2),
**characterised in that**,
the device has means (4, 5, 6) for detecting the spatial position of the treatment instrument (1), wherein, in a control mode, the detected spatial position of the treatment instrument (1) can be converted by the control device into coordinates for controlling a pointer (3) on the display unit (2), wherein the means (4, 5, 6) for detecting the spatial position of the treatment instrument (1) are further constructed in such a way that they also detect a spatial orientation of the treatment instrument (1), the control unit also taking into account the detected spatial orientation and/or alterations of the spatial orientation for the generation of control signals.

2. Dental device according to claim 1,
**characterised in that**,
the means (4, 5, 6) for detecting the spatial position of the treatment instrument (1) on the display unit (2) have at least one infrared light source (5) as well as an optical sensor (6), and at least one reflector (4) mounted on the treatment instrument for reflecting the radiation emitted by the at least one infrared light source (5).

3. Dental device according to claim 1,
**characterised in that**,
the means (4, 5, 6) for detecting the spatial position of the treatment instrument (1) have at least one infrared light source mounted on the treatment instrument (1), as well as an optical sensor (6) arranged on the display unit (2).

4. Dental device according to claim 2 or 3,
**characterised in that**,
the optical sensor is an infrared-sensitive camera (6).

5. Dental device according to claim 2 or 3,
**characterised in that**,
the optical sensor is a 4-field photodiode.

6. Dental device according to any preceding claim,
**characterised in that**,
it has means for generating additional control signals for the pointer (3) during the control mode.

7. Dental device according to claim 6,
**characterised in that**,
it has a switch device (8) mounted on the treatment instrument (1) for generating the additional control signals.

8. Dental device according to claim 6,
**characterised in that**,
it has a foot switch (10) for generating the additional control signals.

9. Dental device according to any preceding claim,
**characterised in that**,
the device for generating further control signals has a speech recognition module.

10. Dental device according to any preceding claim,
**characterised in that**,
the device has a switch (8, 10) for activating the control mode.

11. Method for controlling a dental device,
which has at least one treatment instrument (1), a central control device and a display unit (2),
**characterised in that**,
in a control mode, the spatial position of the treatment instrument (1) is detected and converted into coordinates for controlling and operating a pointer (3) on the display unit (2),
wherein in the control mode in addition to the position there is also detected an orientation of the treatment instrument (1), and the orientation and/or alterations of the orientation are converted into additional control signals.
